# EUROPEAN PATENT APPLICATION

(11) **EP 4 803 025 A1**
(43) Date of publication of application: **09.09.2026**
(21) Application number: 25161680.1
(22) Date of filing: 04.03.2025
(51) Int. Cl.: A61B 34/10, G06F 30/23, A61F 2/46

(54) **COMPUTERIZED SYSTEM AND METHOD TO DETERMINE A VALUE OF A PARAMETER REPRESENTATIVE OF A MECHANICAL INTERACTION OF A SURGICAL SYSTEM WITH AN ANATOMICAL PORTION OF A PATIENT, AND COMPUTER PROGRAM**

(71) Applicant: Spinevision, 92160 Antony (FR)
(72) Inventor: DUCHENES, Renaud, 69500 Bron (FR); DUMESNIL, Hugo, 91370 Verrière-le-Buisson (FR); ROUHBAN, Antony, 75011 PARIS (FR)
(74) Representative: Fidal Innovation

(57) **Abstract**

The computerized system comprises a computerized generation module (36), which generates a finite element model of a surgical system designed to assess the mechanical interaction with an anatomical portion (3) of a patient, a computerized reception module (24), which receives, from the surgical system, signals representative of this mechanical interaction, a computerized adaptation module (27) which adapts the finite element model from said signals, to generate an updated finite element model, and a computerized solver (25) which determines a value of a parameter representative of the mechanical interaction from the updated finite element model.

## Description

### FIELD OF THE INVENTION

The present invention relates to computerized systems and methods to determine a value of a parameter representative of a mechanical interaction of a surgical system with an anatomical portion of a patient, and associated computer programs.

### BACKGROUND OF THE INVENTION

Treatment of spinal disorders, such as degenerative diseases, fractures, scoliosis or other curvature abnormalities, often requires surgical treatments.

A surgical treatment typically involves the use of longitudinal connecting implants, such as rods or plates. Such longitudinal connecting implants can be attached to two or more vertebrae to treat spinal disorders. Longitudinal connecting implants can be used to adjust the relative position of two or more vertebrae and/or can be used to form a stable spine to promote bone fusion.

Anchoring implants can be used to secure the rods to the vertebrae. In particular, these anchoring systems may include anchoring screws, such as pedicle screws, cables, sublaminar bands or hooks. The body of an anchoring implant is fixed in a vertebra, while the head of the anchoring implant, which corresponds to the tulip of an anchoring screw or a hook for example, emerges from the vertebra. The longitudinal connecting implant can then be housed in the head of the anchoring implants. Locking elements can be used to permanently fix the longitudinal connecting implant in the head of the anchoring implant. These locking elements may include clips, a clamping screw, etc.

It might be useful to assist the surgeon during surgery. This might make the surgery easier and more efficient to perform by the surgeon, and/or mitigate per-operative and/or post-operative surgery risks such as pulling out of anchoring implants, rupture of the implants and/or spine adjacent segment changes or disease.

In view of the very broad variability of patients and diseases, pre-operative planning has been introduced, for example to pre-operatively decide key aspects of the surgery. These key aspects could for example be the three-dimensional shape and/or mechanical properties (stiffness) of a longitudinal connecting implant, the position of the anchoring implants, or many other factors.

For example, WO 2023/170,012 defines a mechanical system comprising both the patient and implant candidates. For a plurality of implant candidates, respective mechanical systems are defined. It uses a finite element solver on models of these candidates, to determine the most suitable type of pedicular screws for the planned surgery.

Pre-operative planning is believed to be beneficial so that the surgeon can be assisted to pre-operatively determine the implant strategy that will be implemented during the surgery. The implant strategy may involve, for example, selection of types of medical devices, such as types of anchoring implant, type of longitudinal implants, positioning of these implants on the spinal anatomy and configurations, instrumentation levels, etc... However, due to the variability mentioned above, the surgeon will review and assess per-operative conditions that may influence the pre-operative strategy. There is also a risk that, even though surgery occurs as planned, unplanned post-operative complications arise.

The invention thus aims at mitigating these risks.

### BRIEF SUMMARY OF THE INVENTION

Thus, the invention relates to a computerized system comprising:
- a computerized generation module, adapted to generate at least a finite element model of at least one surgical system designed for mechanical interaction with an anatomical portion of a patient,
- a computerized reception module, adapted to repeatedly receive, from said at least one surgical system, signals representative of said mechanical interaction,
- a computerized adaptation module adapted to adapt the finite element model from said signals, thereby generating an updated finite element model,
- a computerized solver adapted to determine a value of a parameter representative of said mechanical interaction from said updated finite element model.

Thanks to these provisions, accurate finite element simulation results can be taken into account.

According to different aspects, it is possible to provide the one and / or the other of the characteristics below taken alone or in combination.

According to one embodiment, the surgical system comprises an implantable device attachable to the anatomical portion, and a non-implantable surgical tool adapted to attach said implantable device to the anatomical portion, and the computerized reception module is adapted to receive signals from either one or both of the implantable device and the surgical tool.

According to one embodiment, the computerized reception module is adapted to receive signals from the implantable device.

According to one embodiment, the computerized reception module is adapted to receive signals from the surgical tool.

According to one embodiment, said signals received from the surgical tool comprise a measured force applied by the surgical tool.

According to one embodiment, the implantable device is chosen among a spinal rod, a spinal implant, an implantable screw, an implantable bolt, an implantable fixation, an implantable hook, an implantable plate.

According to one embodiment, the computerized generation module is adapted to generate a finite element model further comprising a finite element model of said anatomical portion of a patient.

According to one embodiment, said computerized generation module is adapted to generate said finite element model of an anatomical portion of a patient based on information about the patient.

According to one embodiment, the parameter comprises one or more of an instant or future stress or strain of a part of the surgical system, of the anatomical portion and/or of an interface between the surgical system and the anatomical portion.

According to one embodiment, said computerized reception module is further adapted to repeatedly receive position information representative of the position of the surgical system and/or the anatomical portion, and wherein the computerized adaptation module is adapted to adapt the finite element model also based on this position information.

According to one embodiment, the computerized system further comprises an MMI module adapted to inform a user about said value.

According to one embodiment, the computerized system further comprises a computerized designer module adapted to determine an adapted surgical system based on an output of the computerized solver.

According to one embodiment, the computerized adaptation module is a learned module.

According to one aspect, the invention relates to a computer program comprising instructions which, when the program is executed by a computer, cause this computerized system to execute the steps of the following method :
- repeatedly receiving from said at least one surgical system, at the computerized reception module, signals representative of said mechanical interaction,
- adapting, at the computerized adaptation module, the finite element model from said signals, thereby generating an updated finite element model,
- determining, at the computerized solver, a value of a parameter representative of said mechanical interaction from said updated finite element model.

According to another aspect, the invention relates to a computerized method comprising, with at least a finite element model of at least one surgical system designed for mechanical interaction with an anatomical portion of a patient, generated by a computerized generation module:
- repeatedly receiving from said at least one surgical system, at a computerized reception module, signals representative of said mechanical interaction,
- adapting, at a computerized adaptation module, the finite element model from said signals, thereby generating an updated finite element model,
- determining, at a computerized solver, a value of a parameter representative of said mechanical interaction from said updated finite element model.

According to another aspect, the invention relates to a finite element model of at least one surgical system designed for mechanical interaction with an anatomical portion of a patient, wherein the finite element model was adapted based on signals representative of said mechanical interaction received from said surgical system.

### BRIEF DESCRIPTION OF THE DRAWINGS

Embodiments of the invention will be described below with reference to the drawings, described briefly below:
Fig. 1 schematically represents a surgery room.
Fig. 2 schematically represents a digital twin of an anatomical portion of the patient and of a surgical system.
Fig. 3 is a schematic representation of a stage of spinal surgery.
Fig. 4 schematically represents one step of one example of a surgery.
Figs. 5 and 6 schematically represent an MMI.
Fig. 7 schematically represents a surgical plan.
Fig. 8 schematically shows a computerized system.

In the drawings, identical references designate identical or similar objects.

### DETAILED DESCRIPTION OF THE INVENTION

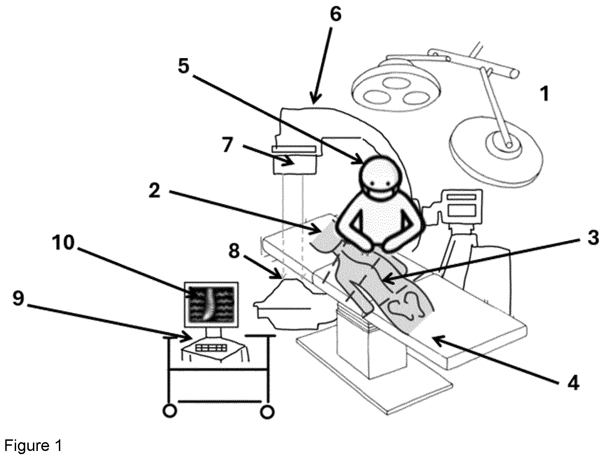

Fig. 1 represents schematically a surgery room 1, where a patient 2 is submitted to surgery. In the examples that follow, reference is made to spine surgery, and the anatomical portion 3 of the patient is its spine, or part of its spine. However, the invention is deemed applicable to other surgeries of other anatomical portions, in particular in the field of orthopedy.

Generally speaking, one may define a frame of reference OXYZ in the surgery room, which will be used and shared by all components. Typically, Z will represent the vertical direction. By convention, X may represent the longitudinal direction of the surgery table, or the main direction of the spine of the patient.

During surgery, the patient 2 is lying on a table 4, so that a surgeon 5, or other medical staff, can access the anatomical portion.

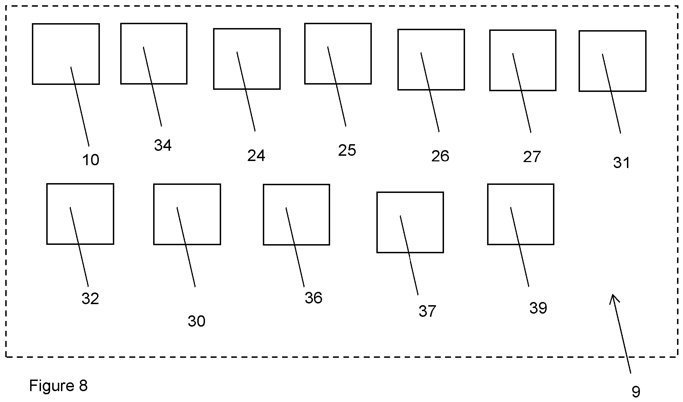

The surgery room 1 may further comprise an imaging system 6, adapted to image the patient, in particular its anatomical portion 3. Typically, the imaging system 6 may be an X-ray imaging system comprising an X-ray source 7, an X-ray detector 8, with the patient located between the source 7 and detector 8, and a computerized system for treating the detected signals and having a display located in the surgery room 1 to display the acquired images. The details of the computerized system according to various embodiments are given on Fig. 8.

The imaging system 6 may be operated upon command and/or continuously.

According to the present example of surgery, one defines as "implantable device", or "implant", a device which is designed to be mechanically attached to an anatomical portion of the patient. This attachment may be direct or indirect, meaning the indirectly-attached device is attached to a device which itself is directly or indirectly attached to the anatomical portion.

One defines as "tool" a device which is not to remain attached to the patient after surgery, and which mechanically interacts with implant(s), either directly or indirectly, during the surgery. By "mechanical interaction", it is meant that force is applied by the surgeon to the implant through the tool, and/or that reaction force exerted by the patient on the implant or tool is transmitted back to the surgeon through the tool.

The set of implants and tools used by the surgeon to perform surgery on the patient is called a "surgical system". For example, the imaging system 6 has no mechanical interaction with the surgeon or patient, and is not considered part of the "surgical system" according to the invention.

According to one embodiment, it is provided a computerized system 9. On Fig. 1, the computerized system 9 is shown in the surgery room 1. However, the computerized system may be provided partly outside of the surgery room 1, and may be distributed over a plurality of computerized devices communicating with one another through network connections.

The computerized system 9 comprises a computerized storage 10. The computerized storage 10 stores a finite element model. The finite element model comprises a finite element model of the components of the surgery which are to mechanically interact with one another during the surgery. In particular, the finite element model may comprise a finite element model of the patient, or of part of the patient, and a finite element model of the surgical system, or of part of the surgical system, as well as a model of the interaction rules between the patient and the surgical system.

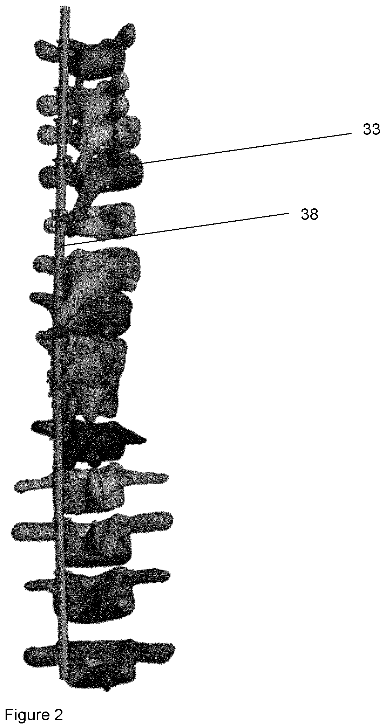

Fig. 2 schematically represents parts of the geometrical aspects of a digital twin of the system constituted by the patient and the surgical system. The digital twin may be tailored for the application. In the present example of spine surgery, the digital twin focuses on parameters relevant for the spine surgery, such as the geometry and mechanical properties of relevant bones and adjacent tissues and of the surgical system. In the present example, there is no digital twin of the head, upper or lower limbs nor of soft tissues, as these are believed to have limited impact on the spine surgery. For example, here, the digital twin will focus on an anatomical portion comprising the vertebrae, the pelvis and the ribcage, as well as soft tissues relevant for ensuring the connection between these bony structures, such as muscles, intervertebral disks and ligaments.

The digital twin of the patient comprises a finite element model of the patient. The finite element model of the patient comprises the geometry of the patient, or, as mentioned, of the relevant anatomical portion of the patient. The geometry may be defined as a so-called "mesh" 33, i.e. a group of one-dimensional, two-dimensional and/or three-dimensional geometrical elements, characterized by the location in space of their vertices (also-called "nodes").

The geometry of the patient may have been determined pre-operatively. For example, pre-operatively, images of the patient are acquired, and the patient-specific mesh is constructed from the pre-operative images. For example, the pre-operative images include images of the internal structure of the patient, such as computer tomography scans (CT-scans), X-rays, and/or even ultrasound, MRI or PET images of the patient. Alternatively or in addition, the pre-operative images may include photographs of the patient.

Alternatively, the three-dimensional geometry of the patient is determined per-operatively directly from the output of the imaging system 6.

The finite element model of the patient further comprises mechanical properties of a material which determine the mechanical behaviour of the materials composing the finite element model. For example, the finite element model of the patient may comprise mechanical properties of a material selected among a library of materials. Mechanical properties of a material may for example include characteristics of elasticity, of plasticity, of visco-elasticity, of poro-elasticity, of compressibility, etc... The mechanical properties of a material may comprise one or more parameters linking deformation to stress, such as elasticity parameters, such as Young's modulus of the material, Poisson ratio, or other parameters; plasticity parameters, such as elastic limit, or failure strain, or other parameters; visco-elastic parameters or the like. One set of material characteristics, a material rule, could be applied to a given material. For example, cortical bone should be defined as an elastic material. Intervertebral disks may be defined as an uncompressible material, etc... For example, all anatomical structures of a same given type of the patient may follow the same deformation rule. The rules may be defined to be patient specific. For example, the finite element model of the patient comprises a patient-specific rule ruling elasticity of its cortical bone. This patient-specific rule may be defined based on patient-specific parameters, such as for example its age, its bone mineral content, determined in Hounsfield Units or DEXA, or pre-operatively determined biomechanical features, such as spine flexibility.

Further, specific rules may be defined for some specific anatomical parts of the patient. For example, various intervertebral disks may be attributed different mechanical rules.

The finite element model of the surgical system comprises the geometry of the surgical system, or, as mentioned, of part of the surgical system. The geometry may also be defined as a so-called "mesh" 38.

The geometry of the surgical system may have been determined pre-operatively. For example, the mesh of a medical device may be provided by the supplier of that medical device. For example, during pre-operative planning, the surgeon chose the type and location of screws he wants to use. The type may include various parameters such as the type, mono- or poly-axial, the length, the material and/or the diameter, or other characterising parameter. Further, the location of the screws is defined in the frame of reference of the surgery room.

Further, the surgeon designed the shape of one or more longitudinal implants (or rods) he wants to implant on the patient. The description below will be done for one rod, but surgery may involve more than one rod, for which the invention is also applicable. The patient-specific rod may have been prepared for the surgery. Such a preparation may involve plastic deformation of an initial rod, in order to achieve the desired patient-specific required geometry. The geometry of the thus deformed rod may be acquired, for example by a stereoscopic imaging system (not shown), in order to digitalize the geometry of the digital twin of the rod. This geometry is also defined in the OXYZ frame of reference of the surgery room.

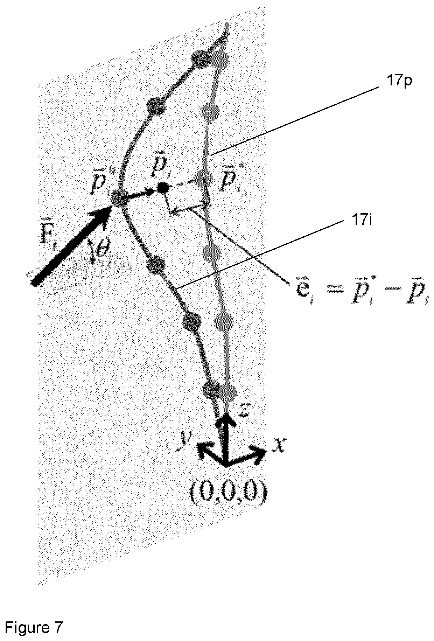

During planning and/or as often as deemed necessary during surgery, the surgeon may also define the osteotomy planning, i.e. the sequence of surgical acts which will start from the uncorrected patient and will finish with the patient with its implants implanted. Fig. 7 shows an example of an embodiment of the invention. According to this embodiment, the former shape and position of the rod is shown by reference 17i, and the later shape and position of the rod is shown by the reference 17p. The surgery, or part of the surgery, consists in passing the rod from its former shape and position 17i to a later shape and position 17p. A sequence of surgical acts to be performed along time to pass the rod from its former shape and position 17i to a later shape and position 17p is defined. This involves for example fixing a set of screws in a given order, or even defines a succession of fixation steps to be applied to the screws. In the example of Figure 7, it involves, as a next step, applying a force indicated by Fᵢ, under an orientation indicated by θᵢ, on a given point, indicated by pᵢ.

The finite element model of the surgical system further comprises material rules which determine the mechanical behaviour of the materials composing the finite element model. For example, the finite element model of the surgical system may comprise material rules selected among a library of materials. Material rules may for example include rules of elasticity or of plasticity, of visco-elasticity, of compressibility, etc... One rule could be applied to a given material. For example, stainless steel or the used titanium alloy should be defined as an elastic-plastic material. Intervertebral disk implants may be defined as an uncompressible material, etc...

The finite element model of the system made of the patient and the surgical system should also comprise rules ruling the interactions of the finite element model of the patient and of that of the surgical system. Such rules are for example contact rules, which define the interaction of two components when they come in contact (transfer of constraints and movements from one component to the other component).

The computerized system 9 thus comprises a computerized generation module 36 adapted to generate the finite element model based on the available information stored in the computerized storage 10.

It should be noted that, during pre-operative planning, the surgeon may use finite element simulation in order to determine the above variables, such as implant features and/or sequence of surgical acts.

Any other relevant data may be used for pre-operative planning as needed. For example, some features of the patient may be monitored before surgery, such as general health parameters or activity parameters, and these features are used for the pre-operative planning.

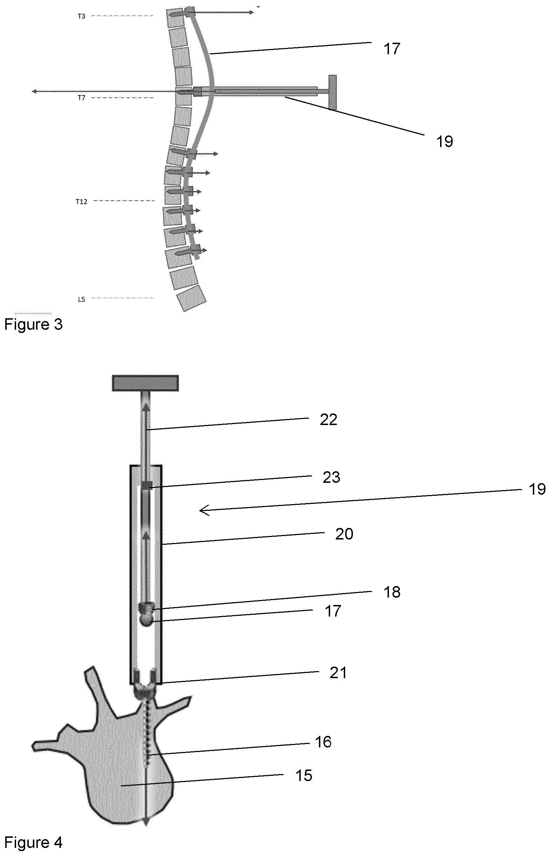

Figs. 3 and 4 give a more detailed example of a step of the surgery. Fig. 3 is a schematic view of the actual stage of the surgery, with a rod 17 having been attached already to vertebrae T3, T9, T10, T11, T12, L1 and L2, and the next step of the surgery being attachment of the rod to vertebra T7. As can be seen on figure 4, the anatomical portion of the patient comprises a vertebra 15 which, in this particular example, is vertebra T7. Previously during the surgery, a pedicular screw basis 16, i.e. the basis of an implant, has been inserted (screwed) into the vertebra 15. The figure 4 also features the rod 17, i.e. a second implant, to be attached to the vertebra 15 T7 through screws. The rod 17 is shown in cross-section in its original location. The figure 4 also features a screw cap 18. The screw cap 18 is represented remote from the screw basis 16, but is designed to be assembled to the screw basis 16 to form, together with it, a screw, so that the rod 17 is retained between the screw basis 16 and the screw cap 18.

The figures 3 and 4 further feature a surgical tool 19. The surgical tool is used by the surgeon during the surgery, but is not an implant, and is not designed to remain into the patient after surgery.

In particular, the surgical tool 19 is designed to assist in the fixation of the implants to the anatomical portion of the patient.

According to one example, the tool comprises a hollow shaft 20 with an open end 21 extending along the insertion direction of the tool 19. The open end 21 is removably assembled to the screw basis 16. The tool 19 comprises a mechanism 22 actuatable to move the screw cap 18 along the hollow shaft 20 in the direction of the open end 21 and hence in the direction of the screw basis 16. Movement of the screw cap 18 in turn pushes the rod 17 toward the screw basis 16. During surgery, the mechanism will be actuated until the screw cap 18 locks on the screw basis 16, trapping the rod 17 in between. The rod 17 thus springs back toward its stable position, pulling the screw with it, and hence the vertebra 15 to which it is attached through the screw, and hence the whole spine through the connection of this vertebra 15 with other anatomical components of the spine, such as shown on Fig. 3. The tool 19 will then be removed from the screw basis 16.

According to an embodiment, the tool 19 is instrumented. In particular, the tool 19 comprises a sensor 23 adapted to detect the force applied to it by the screw cap 18. Repeatedly, for example periodically, the sensor 23 sends a signal to a remote computerized reception module 24. For example, the sensor 23 is wired to a back-end of the tool 19 which, in turn, is wirelessly connected to the computerized reception module 24, so as not to hinder the surgeon's practice.

Repeatedly, the computerized reception module 24 receives a signal from the sensor 23. The received signal is representative of the force applied by the screw cap 18 or the rod on the tool 19.

When the surgeon judges it useful, a finite element model of the system is generated by the computerized generation module 36. The finite element model of the system may for example be generated based on a finite element model of the system previously used during the surgery and stored in the computerized storage 10. Alternatively, the finite element model of the system may be generated by assembling various components stored in the computerized storage 10. In this example, the finite element model will be generated based on the finite element model of the patient and of the finite element model of the surgical system.

The features of the model may be adapted to reflect the current status of the surgery. Because the position of the patient in the surgery room is not necessarily the same as his position during pre-operative imaging, the computerized system 9 may comprise a computerized alignment module 34 adapted to adapt the pre-operatively-obtained geometry to the instant geometry of the patient, as determined by the imaging system 6.

As well, the positions and orientations of the models of the already installed implant(s) may be adapted based on the instant geometry of the model of the spine.

The position, orientation and shape of the model of rod may be adapted from the initial model to reflect the previous steps of the surgery.

The finite element model may further comprise limit conditions, such as for example a definition of some points of the finite element model where displacements are constrained, such as for example points which are considered of fixed location, or predetermined degrees of freedom, such as pivot, slide, sliding pivot, planar joint, parallel cylinders, cylindrical, spherical, edge slider, cylindrical slider, crossed cylinder, etc...

The finite element model may further comprise definitions of connection forces between parts of the model, in particular the definition of forces between vertebrae and screws already attached to the vertebrae.

If the finite element model is generated based on a previous use of the model during surgery, some of these parameters may be already determined, and only few parameters have to be modified to get to a finite element model accurately reflecting the actual condition.

The computerized system 9 further comprises a computerized solver 25 adapted to determine modelled fields of deformation, or strain, and of stress, for the finite element model.

Hence, the computerized solver 25 is run for the finite element model generated by the computerized generation module 36. The computerized solver 25 may be run according to one or more modes. For example, the computerized solver 25 may be run in quasi-static or dynamic mode. The computerized solver 25 may be run in small displacements or large displacements mode. The computerized solver 25 may be run in small strain or large strain mode. In particular, in the finite element model, according to a first embodiment, and as shown on Fig. 7, a displacement corresponding to the displacement applied to the screw cap by the surgical tool is defined. In particular, the computerized solver 25 determines a modelled force for the interaction of the screw cap and the tool in the model in response to the applied displacement. Of course, the whole modelled mechanical system is taken into account by the computerized solver 25, in particular the fact that, at that time, the rod 17 is attached already to at least one other vertebra.

The computerized system 9 comprises a computerized validation module 26 adapted to validate the finite element model. Indeed, the finite element model, as presented above, is built on assumptions. During the previous surgical step, the surgeon applied the same displacement to the real rod, using the surgical tool, by way of the screw cap, as the displacement entered in the model, and the position and shape of the real rod at the end of this surgical step is acquired. The computerized validation module 26 uses the signal received by the computerized reception module 24 to validate the finite element model. In particular, the computerized validation module 26 compares the signal received by the computerized reception module 24 with the determined amplitude of the modelled force for the interaction of the screw cap and the tool in the model to validate the finite element model. For example, if the difference between the signal received by the computerized reception module 24 and the determined amplitude of the modelled force for the interaction of the screw cap and the tool in the model is less than a predefined threshold, the finite element model is validated. Indeed, it means that the force estimated by the model is also measured during the real surgery.

However, the computerized validation module 26 may fail to validate the finite element model. This would be the case, for example, if the difference between the signal received by the computerized reception module 24 and the determined amplitude of the modelled force for the interaction of the rod 18 and the tool 19 in the model is greater than the predefined threshold. This might trigger an alert on the fact that the model has a discrepancy with reality.

In such case, a computerized adaptation module 27 is adapted to adapt the finite element model. More precisely, the computerized adaptation module 27 is adapted to adapt the finite element model into an updated finite element model. An adaptation algorithm is running until the computerized validation module 26 validates the updated finite element model.

The computerized adaptation module 27 is adapted to adapt the finite element model by changing one or more of the parameters of the finite element model.

The one or more parameters include for example one or more of the following parameters:
- the number of finite elements,
- a parameter regarding the limit conditions, such as for example the number and/or location of nodes with specific degree of freedom conditions,
- the type of elements, such as, for volumic elements, tetrahedrons or hexahedrons,
- the type of interpolation function of elements, such as linear, quadratic or cubic,
- a parameter relating to the applied forces, such as for example the number and/or location of force application nodes, the type of force, the orientation and/or amplitude of the force,
- a parameter related to one or more interfaces between components of the model, such as the number and/or location of nodes involved in a contact between two components, and/or a friction coefficient,
- a parameter related to constraints, such as the number and/or locations of involved nodes and/or orientation of these constraints,
- the time step,
- the solver,
- the integration scheme, such as direct or indirect integration,
- the position and/or orientation of one or more anatomical portions, and/or one or more surgical systems,
- the position and/or orientation of one or more forces applied to one or more anatomical portions,
- the timing of the previous steps of the surgery, such as the timing of the applications of forces, the timing of assembling and/or tightening implants to vertebras, the identity of the anatomical portion to which tightening was first exerted, the times at which forces were exerted or stopped being exerted,
- the type of constraints applied between two elements, such as for example the application of tightening between an anatomical portion and an associated surgical implant,
- any material parameter or rule.

The computerized solver 25 solves the updated finite element model, and compares the modelled amplitude value for the force for the interaction of the screw cap, the rod and the tool in the updated model with the measured signal. As mentioned above, at this stage, the computerized validation module 26 may validate the updated finite element model. But if, at this stage, the computerized validation module 26 does not validate the updated finite element model, the modelled value determined by the updated model may be compared to previously determined value(s) of previous models and to the measured signal, in order to determine whether the updated model is converging toward the real life solution. If the amplitude of the modelled value determined by the updated model is getting closer to the measured signal than that of the previously obtained modelled values, it may mean that the updated model is getting closer to validation, and this may guide future adaptations of the model. On the contrary, if the amplitude of the modelled value determined by the updated model is getting farther away from the measured signal than the previously obtained amplitudes of the modelled values, it may mean that the updated model is getting farther away from validation, and this may guide future adaptations of the model.

Alternatively, other options are possible for validating the finite element model. For example, the force measured at the tool 19 in input in the finite element model, and the displacement determined by the model is compared to the actual displacement performed during surgery when applying this force. The finite element model is adapted, as described above, until the modelled displacement matches the actual displacement. In such case, the parameters of the model which may be modified for validation of the model may include the force profiles along time or the maxima of the applied forces.

Once the finite element model is validated, the computerized solver 25 solves the finite element model, in order to determined modelled stresses and/or strains in the finite element model. Alternatively, the results of the latest validation step are used. The results of the simulation may be used by the surgeon to adapt the surgery, and/or adapt the use of tools or implants used for the surgery. For example, the modelled stresses or strains may be compared to predefined threshold values, and an alarm may be emitted in case a modelled stress or strain goes beyond a predefined threshold value. For example, it is determined that a stress at another screw exceeds a predefined threshold value, which, in turn would indicate a risk of pulling out of that screw from the vertebra it is screwed in. This would allow the surgeon to step back, and to modify the sequence of surgical steps.

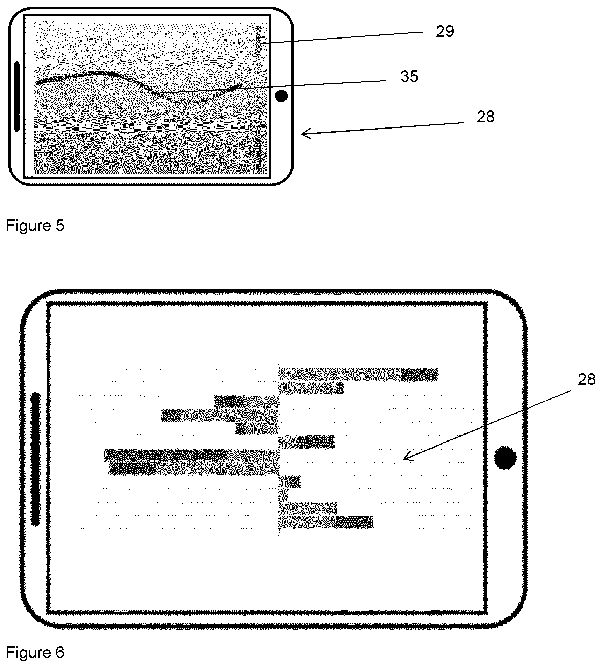

Fig. 5 gives an example of the result provided by the computerized solver 25 for the rod 17 at the present stage of the surgery. The result is provided on a man-machine interface module 28, for example comprising a display, of the computerized system 9. The display comprises a scale 29 of stresses, for example a color scale, and a 3D representation (deformed mesh) 35 of the rod colored according to the locally modelled stresses and according to the scale 29. This allows the surgeon to visualise any relevant biomechanical criteria, such as the location and/or intensity of the highest or lowest stresses.

Alternatively or in addition, the MMI 28 may provide information such as the modelled force at the interface between the vertebra and each respective screw, such as shown on Figure 6. Further, in case a pre-operative simulation was performed, during which the maximum of the forces exerted between each screw and the corresponding vertebra were registered, the force actually determined by the model may be compared to this predicted maximum force, as shown. This enables to visualize in particular that all of the actual forces (as determined by the calibrated model) are lower than those predicted during the pre-operative planning.

The criteria above might be either related to one or more implant, as discussed in relation to figure 5, to a modelled interface between an anatomical structure and an implant, as discussed in relation to figure 6, or to one or more anatomical structure. For example, the solver calculates a stress at a bone which goes beyond the strength of the bone, which might be an indication of a risk of failure of the bone. For example, the solver calculates a force at a bone/implant interface which goes beyond a pre-defined force the interface is considered to be able to withstand, which might be an indication of a risk of screw pull-out.

In some embodiments, the computerized system 9 may thus comprise a computerized designer module 99. The computerized designer module 99 can be used during surgery by the surgeon to design a modified implantable device, which would be deemed more suitable to limit the above-determined risks. For example, the surgeon may use the computerized designer module 99 to modify the design of the rod. The rod itself would then be modified according to this design, and the new shape of the rod will be used in the finite element model. Then, surgery can be resumed.

For example, the above steps are performed only once, when the surgeon believes that the finite element model might be useful for a next step of the surgery. According to another example, the above steps may be performed occasionally during surgery, each time the surgeon believes it would be beneficial. According to yet another example, the above steps are repeated for each step of displacing the screw cap 18 and/or the rod toward the screw basis 16, until the screw cap 18 locks to the associated screw basis 16. At this stage, the surgical tool 19 might be withdrawn. For each step, model parameters, such as the starting geometry, may be based on the ones resulting from the previous step.

Then, the surgical process is repeated for another screw cap.

The process can thus be repeated until the surgery is complete.

The example above is a simplified example, where it is described that screws are fully secured and the rod tightened one by one. However, according to another embodiment, the surgeon may use a plurality of surgical tools at the same time, each dedicated to a respective screw. It means that, at a given stage, the computerized reception module receives more than one signals, which may be used as described above. Hence, at a given step, in view of the field of deformations or stresses provided by the validated finite element model, and/or in view of the received signals, the surgeon may decide which tool(s) to actuate as a next step of the surgery.

In an alternative embodiment, one or more surgical tools might be robotically actuated. The force sensor might then be integrated in the robot, and/or the robot may provide additional or alternative signals related to the mechanical interaction of the surgical tool with the patient. For example, a signal may be related to characteristics of an electric current used to drive the robot to perform an actuation during a surgical step.

Thus, the latest simulation will provide a full and accurate biomechanical characterisation of the patient. It may provide one or more of the following features: a clinical feature, a clinical feature of the spine, such as the Cobb angle of the patient's scoliosis, one or more kyphosis/lordosis angle of the patient, one or more pelvic parameters, the three dimensional shape of one or more rods, or other clinical features; a biomechanical feature, such as one or more stress or load between the screws and the rod, one or more stress or loads on one or more screw, one or more stress or loads on the rod, areas of plastic deformation of the rod(s), or loads along the spine, or other biomechanical features, or other parameters of interest.

According to one embodiment, the simulation may trigger at any time an alert, based on the validated finite element model, that the current set of implants or implant locations will not be suitable for the planned surgery. This would allow the surgeon to change the implant(s) or implant location(s) during the surgery.

It should be mentioned also that, in alternative to the embodiment described above, some or all steps which are described as pre-operative may be implemented intraoperatively, namely at the start of the surgery, for example with the patient in position for surgery.

The patient may be monitored after surgery.

The computerized system 9 might comprise a computerized virtual activity module 31. The computerized virtual activity module 31 comprises the latest finite element model of the patient with the implanted implants, and is adapted to determine the biomechanical response of the patient to one or more activities, which can be simulated using this module. For example, one activity is the activity of standing. Under this activity, the patient is represented standing (whereas the surgery is performed with the patient lying), and the biomechanical effect of gravity is determined using the finite element solver 25. The result of the simulation may be compared to pre-determined rules in order to check whether the patient with its implants can support the activity. Other conditions of loading might be defined in the computerized virtual activity module 31, such as walking, running, jumping, etc... The virtual activity module 31 may also use input data received from an activity sensor (not shown), such as one or more accelerometer or goniometer worn by the patient. This input data may be used by the virtual activity module 31 to determine an actual activity by the patient. For example, based on this input data, the virtual activity module 31 determines that the patient is running, and performs a simulation for this activity.

In the above embodiment, the surgical tool is instrumented. Alternatively, or in addition, an implant might be instrumented. For example, instead of the surgical tool 19, the screw cap 18 is instrumented. The screw cap 18 is designed to communicate wirelessly with the computerized reception module 24. Hence, the above example can be completed or modified in that the signal from the screw cap 18 is compared to the modelled value of the force between the rod 17 and the screw cap 18. This brings the additional benefit that signals continues to be received even upon withdrawal of the a surgical tool 19.

According to one embodiment, the instrumented implant may continue to communicate signals to a computerized reception module 24 after surgery. For example, a computerized reception module 24 may be part of an electronic system (not shown) borne by the patient, such as a smart watch or a smartphone or the like. This electronic system may comprise, or communicate with a computerized analysis module 32 adapted to analyse said signals. For example, the computerized analysis module 32 may compare the received signal with a predetermined threshold regarding the amplitude of the rod-screw cap interaction force, and may issue an alert to the patient or the surgeon in case this comparison reveals an alert should be emitted.

In addition, this signal may be sent to the computerized adaptation module 27 in order to determine an updated model based on the latest received signal. In turn, the computerized solver 25 may run a simulation based on the thus adapted model as part of patient monitoring. This may enable to determine a concern in the biomechanical system, especially at a location remote from the sensor, for example a local stress exceeding a predefined threshold. The computerized solver 25 may issue an alert to the patient or the surgeon in case this comparison reveals an alert should be emitted. However, the computerized solver 25 may remain a component used during surgery only. Thus, according to one embodiment, the computerized system 9 may comprise a computerized post-op monitor 39 comprising a finite element solver to run simulations for post-op patient monitoring, such as was described above.

All data, either modelled or measured, during surgery, may be stored in a computerized storage 30, such as a hard disk or the like.

According to one example, the computerized generation module is a learned module.

According to this example, it is provided a computerized learning module, adapted to design the learned computerized generation module.

As an input to the learning module, it is provided, for each patient, data at the beginning of the surgery, also called "initial data", as well as data at the end of the surgery, also called "final data". Initial data includes for example patient-specific geometry and pre-operative planning (in terms of intended implants and their initial and final geometry and position). "Final data" includes for example the parameters of the finite element model at the end of the surgery.

The computerized generation module is taught to provide the best match between the initial data and the final data for each patient of the training data set. What is a "best match" may be defined in any suitable way, such as by an average distance between the final data and the outcome of the learned computerized generation module for the corresponding initial data.

According to the example below, the "initial" and "final" data refer to the whole surgery. However, a computerized generation module may be learned for each or some individual surgical steps. In such case, as an input to the learning module, it is provided, for each patient, data at the beginning of the surgical step, also called "former data", as well as data at the end of the surgical step, also called "later data".

According to one example, the computerized adaptation module is a learned module.

According to this example, it is provided a computerized learning module, adapted to design the learned computerized adaptation module.

As an input to the learning module, it is provided, for each patient, data at the beginning of a surgical step, also called "initial data", as well as data at the end of this surgical step, also called "final data". Initial data includes for example patient-specific and implant geometry at the beginning of the given step of the surgery, mismatch between measurement value and modelled force determined by the finite element model, as well as finite element model parameters at this stage. "Final data" includes for example the changes to the finite element model at the end of the step.

The computerized adaptation module is taught to provide the best match between the initial data and the final data of the training data set for the given surgical step. What is a "best match" may be defined in any suitable way, such as by an average distance between the final data and the outcome of the learned computerized adaptation module for the corresponding initial data.

For example, when the surgical step is a further step of persuasion of a screw cap to T12, the learned computerized adaptation module will suggest that, based on training data, the best way to reduce the mismatch between the modelled force and the measured signal is to reduce by 5% the stiffness of the material representing the intervertebral discs in the finite element model.

Thus, one or more of the surgical steps may be learned according to the description above.

In addition, input data for the computerized learning module may be the outcome of the surgery for the patient. For example, the outcome of the surgery might be the information that corrective surgery was necessary for this patient. The outcome of the surgery might be a number of days since the surgery without a corrective surgery being needed.

According to one embodiment, a patient-specific finite element model of the patient might not be necessary. The finite element model will comprise the implants and tools. For example, the location of the implanted implants might be considered fixed or subjected to predefined constraints for the following simulations.

The invention relies on computerized modules. Operation of such modules may involve one or more processors of computers executing software comprising instructions defining the functions of the respective modules.

While exemplary embodiment of the invention has been described with reference to two main embodiments, it will be understood by those skilled in the art that various changes, omissions and/or additions may be made, and equivalents may be substituted for elements thereof without departing from the spirit and scope of the invention. In addition, many modifications may be made to adapt a particular situation or material to the teachings of the invention without departing from the scope thereof. Therefore, it is intended that the invention is not limited to the particular embodiment disclosed as the best mode contemplated for carrying out this invention, but that the invention will include all embodiments falling within the scope of the appended claims. Moreover, unless specifically stated any use of the terms first, second, etc. do not denote any order or importance, but rather the terms first, second, etc. are used to distinguish one element from another.

### LIST OF REFERENCE SIGNS

1 : Surgery room
2: patient
3: anatomical portion
4: table
5: surgeon
6: imaging system
7: X-ray source
8: X-ray detector
9: Computerized system
10: computerized storage
15: vertebra
16: pedicular screw basis
17: rod
18: screw cap
19: surgical tool
20: hollow shaft
21: open end
22: mechanism
23: sensor
24: computerized reception module
25: computerized solver
26: computerized validation module
27: computerized adaptation module
28: MMI module
29: scale
30: computerized storage
31: computerized virtual activity module
32: computerized analysis module
33: mesh
34: computerized alignment module
35: 3D representation
36: computerized generation module
37: computerized designer module
38: mesh
39: post-op monitor

## Claims

1. A computerized system comprising:
- a computerized generation module (36), adapted to generate at least a finite element model of at least one surgical system designed for mechanical interaction with an anatomical portion (3) of a patient,
- a computerized reception module (24), adapted to repeatedly receive, from said at least one surgical system, signals representative of said mechanical interaction,
- a computerized adaptation module (27) adapted to adapt the finite element model from said signals, thereby generating an updated finite element model,
- a computerized solver (25) adapted to determine a value of a parameter representative of said mechanical interaction from said updated finite element model.

2. The computerized system according to claim 1, wherein the surgical system comprises an implantable device attachable to the anatomical portion, and a non-implantable surgical tool (19) adapted to attach said implantable device to the anatomical portion, and wherein the computerized reception module is adapted to receive signals from either one or both of the implantable device and the surgical tool.

3. The computerized system according to claim 2, wherein the computerized reception module (24) is adapted to receive signals from the implantable device.

4. The computerized system according to claim 2 or 3, wherein the computerized reception module (24) is adapted to receive signals from the surgical tool (19).

5. The computerized system according to claim 4, wherein said signals received from the surgical tool (19) comprise a measured force applied by the surgical tool (19).

6. The computerized system according to any of claims 2 to 5, wherein the implantable device is chosen among a spinal rod (17), a spinal implant, an implantable screw, an implantable bolt, an implantable fixation, an implantable hook, an implantable plate.

7. The computerized system according to any of claims 1 to 6, wherein the computerized generation module (36) is adapted to generate a finite element model further comprising a finite element model of said anatomical portion of a patient, notably based on information about the patient.

8. The computerized system according to any of claims 1 to 7, wherein the parameter comprises one or more of an instant or future stress or strain of a part of the surgical system, of the anatomical portion and/or of an interface between the surgical system and the anatomical portion.

9. The computerized system according to any of claims 1 to 8, wherein said computerized reception module (24) is further adapted to repeatedly receive position information representative of the position of the surgical system and/or the anatomical portion, and wherein the computerized adaptation module (27) is adapted to adapt the finite element model also based on this position information.

10. The computerized system according to any of claims 1 to 9, further comprising an MMI module (28) adapted to inform a user about said value.

11. The computerized system according to any of claims 1 to 10, further comprising a computerized designer module (37) adapted to determine an adapted surgical system based on an output of the computerized solver (25).

12. The computerized system according to any of claims 1 to 11, wherein the computerized adaptation module (27) is a learned module.

13. A computer program comprising instructions which, when the program is executed by a computer, cause the computerized system of any of claims 1 to 12 to execute the steps of the following method :
- repeatedly receiving from said at least one surgical system, at the computerized reception module (24), signals representative of said mechanical interaction,
- adapting, at the computerized adaptation module (27), the finite element model from said signals, thereby generating an updated finite element model,
- determining, at the computerized solver (25), a value of a parameter representative of said mechanical interaction from said updated finite element model.

14. A computerized method comprising, with at least a finite element model of at least one surgical system designed for mechanical interaction with an anatomical portion of a patient, generated by a computerized generation module (36):
- repeatedly receiving from said at least one surgical system, at a computerized reception module (24), signals representative of said mechanical interaction,
- adapting, at a computerized adaptation module (27), the finite element model from said signals, thereby generating an updated finite element model,
- determining, at a computerized solver (25), a value of a parameter representative of said mechanical interaction from said updated finite element model.

15. A finite element model of at least one surgical system designed for mechanical interaction with an anatomical portion of a patient, wherein the finite element model was adapted based on signals representative of said mechanical interaction received from said surgical system.
